# EUROPÄISCHE PATENTSCHRIFT

(11) **EP 4 500 477 B1**
(45) Veröffentlichungstag und Bekanntmachung des Hinweises auf die Patenterteilung: **24.12.2025**
(21) Anmeldenummer: 23725587.2
(22) Anmeldetag: 04.05.2023
(51) Int. Cl.: G06T 17/00, A61C 13/00

(54) **VERFAHREN ZUM VISUALISIEREN UND MODELLIEREN VON ZAHNERSATZ**
METHOD FOR VISUALIZING AND MODELING A DENTAL PROSTHESIS
PROCÉDÉ DE VISUALISATION ET DE MODÉLISATION D'UNE PROTHÈSE DENTAIRE

(30) Priorität: 30.05.2022 DE 102022113599; 24.02.2023 DE 102023104576
(43) Veröffentlichungstag der Anmeldung: 05.02.2025
(73) Patentinhaber: Kombo Medical Solutions UG, 38642 Goslar (DE)
(72) Erfinder: KUBIACK, Kim, 38642 Goslar (DE); NITSCH, Carsten, 38642 Goslar (DE)
(74) Vertreter: Scheffler, Jörg
(86) Internationale Anmeldenummer: PCT/EP2023/061808
(87) Internationale Veröffentlichungsnummer: WO 2023/232384

(56) Entgegenhaltungen:
- WO-A1-2013/120478
- DE-A1- 102019 106 666
- US-A1- 2021 153 986
- US-A1- 2021 186 659
- KURT BAYRAKDAR SEVDA ET AL: "A deep learning approach for dental implant planning in cone-beam computed tomography images", BMC MEDICAL IMAGING, vol. 21, no. 1, 19 May 2021 (2021-05-19), XP093076217, Retrieved from the Internet <URL:https://bmcmedimaging.biomedcentral.com/counter/pdf/10.1186/s12880-021-00618-z.pdf> DOI: 10.1186/s12880-021-00618-z
- SONG WEINAN ET AL: "Oral-3D: Reconstructing the 3D Structure of Oral Cavity from Panoramic X-ray", PROCEEDINGS OF THE AAAI CONFERENCE ON ARTIFICIAL INTELLIGENCE, vol. 35, 2 February 2021 (2021-02-02), pages 566 - 573, XP093076195, ISSN: 2159-5399, DOI: 10.1609/aaai.v35i1.16135
- KANG DAE-YOUNG ET AL: "Application of Deep Learning in Dentistry and Implantology", THE KOREAN ACADEMY OF ORAL AND MAXILLOFACIAL IMPLANTOLOGY, vol. 24, no. 3, September 2020 (2020-09-01), pages 148 - 181, XP055943855, ISSN: 1229-5418, DOI: 10.32542/implantology.202015

## Beschreibung

Die Erfindung betrifft ein Verfahren zum Visualisieren und Modellieren von Zahnersatz durch ein virtuelles 3D-Modell des Zahnersatzes, wobei 2D-Röntgenbilder der ursprünglich vorhandenen natürlichen Zähne des Patienten verwendet werden. Weiterhin betrifft die Erfindung ein Verfahren zur Herstellung von Zahnersatz für einen Patienten auf der Basis zumindest eines Datensatzes eines virtuellen 3D-Modells.

Häufig werden in der zahnmedizinischen Praxis durch plastisch aushärtende Abdruck- bzw. Abformmassen Ober- und Unterkiefer eines Patienten abgeformt, um einen Abdruck zu erhalten. Der Abdruck dient als Basis zur Herstellung eines Modells. Auf dem Modell formt ein Zahntechniker den Zahnersatz, der meist als Wachsmodell ausgeführt ist. Das Wachsmodell dient gewöhnlich als Positivform für eine Negativform, aus der in bekannter handwerklicher Art der Zahnersatz gewonnen und anschließend dem Patienten angepasst und eingesetzt wird.

Nach dem Verlust mehrerer Zähne eines Patienten geht für immer die Information über die ursprüngliche Anatomie dieser Zähne und des umgebenden Knochens verloren. Mit dem Zahnverlust geht auch die Information über den ursprünglichen natürlichen Zusammenbiss der Zähne und deren Position und Orientierung zueinander verloren. Diese von Natur aus angelegte Situation des Zusammenbeißens beider Kiefer wiederum verschlüsselt auch eine präzise Lagesituation des Kiefergelenk-Komplexes und lässt sich in diesem Fall nicht mehr ermitteln.

Beim Verlust von mehreren Zähnen gehen diese anatomischen Informationen in unterschiedlichem Maße verloren. Die Information eines einzelnen Zahns in Form und Farbe, die Zahnstellung auch innerhalb der Position gegenüber weiteren Zähnen, die umgebende Kieferform und die Lagebeziehung des Kiefergelenks in der Schlussbissstellung kann nach dem Verlust der Zähne dauerhaft verloren sein.

Die Herstellung von Zahnersatz durch den Zahnarzt und den Zahntechniker hat zum Ziel, diese verloren gegangenen Informationen zu rekonstruieren und den Zahnersatz möglichst als Abbild oder als eine Optimierung der natürlichen Zähne und Kieferanteile zu fertigen.

Aktuelle Verfahren, die dem Stand der Technik entsprechen, basieren auf der Hinzuziehung von Referenzwerten anderer Punkte noch vorhandener anatomischer Strukturen und dem Einsatz von Erfahrungen und theoretischen Kenntnissen des Zahnarztes und Zahntechnikers, um der ursprünglichen Form und Position möglichst nahe zu kommen. Somit ist das Endergebnis weitestgehend offen im finalen Erscheinungsbild. Misst man die Qualität des Zahnersatzes an dem Wert, wie weit der Zahnersatz den ursprünglichen Zähnen entspricht, so sind die bisherigen Verfahren, die dem aktuellen Stand der Technik entsprechen, nicht geeignet, eine vorhersagbare Qualität zu erreichen, da weitestgehend ohne Kenntnis der ursprünglichen Formen gearbeitet wird.

Grundsätzlich gibt es viele Verfahren im Zusammenhang mit der intraoralen Kavität, in denen eine genaue dreidimensionale virtuelle Darstellung der intraoralen Kavität für den praktischen Zahnarzt nützlich sein kann.

Derartige virtuelle Darstellungen (die hierin auch gleichbedeutend als "virtuelle Modelle", "Computermodelle", "numerische 3D-Einheiten" usw. bezeichnet werden) ermöglichen es dem Anwender, die intraorale Kavität bzw. innere Mundhöhle einzelner Patienten über ein Computersystem ähnlich zu der Untersuchung des herkömmlichen mechanischen Gipsmodells zu erfassen.

In der Zahnmedizin sind zur Generierung von medizinischen 3D-Datensätzen dreidimensionale Bildgebungsverfahren, beispielsweise dreidimensionales Röntgen, Kernspintomographie oder Magnetresonanztomographie (MRT), dreidimensionaler Computertomographie (CT) bzw. digitale Volumentomographie (DVT), beispielsweise aus der DE 10 2008 009 643 A1 oder DE 10 2004 035 475 A1 bekannt.

Bei einem Verfahren zur Herstellung von Zahnersatzteilen gemäß der EP 2 010 090 B1 wird ein Messdatensatz einer 3D-Röntgenaufnahme im Bereich der einzusetzenden Prothese bereitgestellt und auf einer Anzeigeeinheit als 3D-Röntgenmodell dargestellt.

Die WO 2012 / 000 511 A1 bezieht sich auf ein Verfahren zum Visualisieren und Modellieren eines Gebisses bei der Zahnrestauration sowie die Bereitstellung eines virtuellen 3D-Modells des Gebisses. Hierzu werden zunächst einerseits 2D-Bilder, beispielsweise Röntgenbilder, mit zumindest einem Gesichtsmerkmal bereitgestellt und andererseits ein virtuelles 3D-Modell von mindestens einem Teil der Mundhöhle des Patienten durch Scannen eines physikalischen Modells oder der Zähne des Patienten erzeugt. Anschließend werden die 2D-Bilder relativ zu dem virtuellen 3D-Modell in einem virtuellen 3D-Raum unter Verwendung detektierter entsprechender anatomischer Punkte zueinander ausgerichtet und im 3D-Raum visualisiert. Darüber hinaus kann ein Zahntechniker eine Restauration auf dem virtuellen 3D-Modell digital entwerfen oder modellieren und dabei Erfahrungen und Kenntnisse über Zahnästhetik und -regeln verwenden.

Ferner ist aus der EP 2 509 507 B1 ein Verfahren zum Verarbeiten eines Röntgenbilds bekannt, wobei eine Ultraschallsonde in dem 2D-Röntgenbild detektiert und die Position und Ausrichtung der Ultraschallsonde in Bezug auf ein Referenzkoordinatensystem geschätzt wird.

In der Praxis hat es sich als nachteilig erwiesen, dass die für eine exakte Rekonstruktion des verlorenen Zahns erforderlichen 3D-Daten zur Herstellung des Zahnersatzes im Gegensatz zu Röntgenbildern häufig nicht verfügbar sind. In solchen Fällen bleibt es häufig dem Geschick des behandelnden Arztes oder des Zahntechnikers überlassen, aufgrund der Erfahrungen eine geeignete Zahnform herzustellen und gegebenenfalls im Rahmen wiederholter Anpassungen zu optimieren.

Dieser Anpassungsprozess ist für den Patienten zeitaufwendig und unbequem. Darüber hinaus hat es sich aber auch gezeigt, dass vor allem bei einem großen Zahnersatz mehrerer oder einer Vielzahl von Zähnen bei den in der Praxis nicht auszuschließenden Abweichungen und Toleranzen Folgeprobleme auftreten, wie beispielsweise Druckschmerzen, Beeinträchtigung benachbarter Zähne, Schmerzen im Gesichts- und Kieferbereich, Knack- oder Reibegeräusche der Kiefergelenke, überdurchschnittliche Abnutzung der Zähne, Nacken- und Rückenprobleme bis hin zu Haltungsschäden. Außerdem kann es zum Lispeln, zu Pfeifgeräuschen oder zu einer Veränderung der Stimme kommen.

Ferner beschreibt die US 2021 / 0 153 986 A1 ein computerimplementiertes Verfahren, ein System und Speichermedien zur Erzeugung dreidimensionaler Zahnersatzgeometrien aus zweidimensionalen Dentalentwürfen mit definierten Designvorgaben. Ein erstes trainiertes neuronales Netz überführt den 2D-Entwurf in eine latente Repräsentation, die ein zweites trainiertes Netz zu einer 3D-Form hochskaliert. Alternativ erfolgt die Umsetzung über eine Übersetzung in Parameter eines parametrischen Modells oder end-to-end über ein 3D-GAN. Die resultierenden Geometrien liegen als Dreiecksnetze oder rasterisierte 3D-Daten vor und werden anhand anatomischer Randbedingungen aus Patienten-3D-Scans zu einer finalen digitalen Restauration angepasst und mittels CAD/CAM hergestellt. Offenbart werden zudem Trainingskonzepte mit Datensätzen aus 2D-Skizzen und zugehörigen 3D-Modellen einschließlich optionaler Nachtrainings auf den Zeichenstil einzelner Nutzer sowie eine Systemarchitektur mit Kamera, Trainingsmodul, Datenbank und CAD/CAM-Komponenten.

Aus der DE 10 2019 106 666 A1 geht zudem ein Verfahren und System zur automatisierten Gestaltung von Zahnrestaurationen mit neuronalen Netzen hervor, die auf Gebissscans trainiert werden. Verwendet werden Datensätze mit realen Präparationsstellen und zugeordneten Technikerprothesen sowie natürliche Gebissscans mit digital erzeugten Präparationsstellen, einschließlich Bogensegmentierung und gegebenenfalls Umwandlung von 3D- in 2D-Tiefenkarten. Aus Patientenscandaten identifiziert ein trainiertes Netzwerk Zahninformationen wie Präparationsgrenzen und erzeugt ein vollständiges 3D Zahnrestaurationsmodell mit okklusalem Abschnitt, Grenzlinie und Seitenwänden unter Einsatz von Klassifikationsnetzen und generativen Netzen einschließlich GAN. Offenbart sind eine verteilte Systemarchitektur mit Servern, Client und Scangeräten sowie Modulen für Training Erkennung und qualitative Bewertung. Beschrieben sind zudem maßgeschneiderte Trainingsdatensätze anhand demografischer und verhaltensbezogener Patientendaten und ein Verfahren zum Verschmelzen von Merkmalen aus unterschiedlich trainierten Modellen in einer Benutzerschnittstelle.

Der Erfindung liegt die Aufgabe zugrunde, dass der nach dem Verfahren hergestellte Zahnersatz keine oder nahezu keine erkennbaren Abweichungen von dem ursprünglichen natürlichen Zahn oder lediglich gewünschte Optimierungen oder Modifikationen aufweist.

Diese Aufgabe wird erfindungsgemäß mit einem Verfahren gemäß den Merkmalen des Anspruchs 1 gelöst. Die weitere Ausgestaltung der Erfindung ist den Unteransprüchen zu entnehmen.

Erfindungsgemäß ist also ein Verfahren vorgesehen, bei dem der Zahnersatz für einen Patienten auf der Basis zumindest eines Datensatzes eines virtuellen 3D-Modells der ursprünglich vorhandenen natürlichen Zähne des Patienten visualisiert, modelliert und/oder hergestellt wird, wobei 2D-Röntgenbilder der ursprünglich vorhandenen zu ersetzenden natürlichen Zähne des Patienten in ein Deep-Learning-Model eingegeben werden, wobei das Deep-Learning-Modell mit 2D-Röntgenbildern von ausgewählten oder zufälligen statistischen Vergleichspersonen und/oder synthetisch generierten, virtuellen 2D-Röntgenbildern und diesen 2D-Röntgenbildern zugeordneten 3D-Vergleichs-Daten zumindest einzelner Zähne oder Mundraumbereiche trainiert wurde,, wobei die den 2D-Röntgenbildern von Vergleichspersonen und/oder synthetisch generierten, virtuellen 2D-Röntgenbildern zumindest zu einzelnen Zähnen oder Mundraumbereichen zugeordneten 3D-Vergleichs-Daten bekannt und/oder in einer Datenbank abgelegt sind, sodass aufgrund einer vergleichenden Betrachtung eine optimale Übereinstimmung der 2D-Röntgenbildern der ursprünglich vorhandenen zu ersetzenden natürlichen Zähne des Patienten mit einzelnen 2D-Röntgenbildern von Vergleichspersonen und/oder synthetisch generierten, virtuellen 2D-Röntgenbildern ermittelt wird und die mit diesen 2D-Röntgenbildern von Vergleichspersonen und/oder synthetisch generierten, virtuellen 2D-Röntgenbildern verknüpften 3D-Vergleichs-Daten als Basis für die Erstellung des 3D-Modells der ursprünglich vorhandenen natürlichen Zähne des Patienten durch das Deep-Learning-Model verwendet werden, wobei bei mehreren Übereinstimmungen die Auswahl aufgrund von statistischen Daten und zusätzlichen Übereinstimmungsmerkmalen erfolgen oder eine Interpolation durchgeführt werden kann.

Erfindungsgemäß kann auf diese Weise der Zahnersatz und/oder ein körperliches Funktionsmodell auf der Grundlage des so geschaffenen virtuellen 3D-Modells als Datensatz in einem automatisierten additiven oder subtraktiven Herstellungsverfahren in körperlicher Form, insbesondere unter Berücksichtigung von Expertenwissen, hergestellt werden.

Erfindungsgemäß gelingt es erstmals, anders als bei den aus dem Stand der Technik bekannten Verfahren, lediglich auf der Grundlage von 2D-Röntgenbildern der ursprünglich vorhandenen natürlichen Zähne des Patienten, also fehlenden 3D-Daten des zu ersetzenden Zahns, ein virtuelles 3D-Modell des benötigten Zahnersatzes zu schaffen und somit die aus dem Stand der Technik bekannten Probleme vollständig zu lösen.

In hoher, vorhersagbarer Qualität wird beispielsweise ausschließlich durch Verwertung alter Panoramaröntgenbilder des Patienten eine Vorlage für den späteren Zahnersatz als 3D-Modell in einem vollautomatischen Prozess errechnet. Dieses 3D-Modell als Vorlage (Template) kann dann mit aus dem Stand der Technik an sich bekannten Methoden in eine CAD-CAM-Software eines Dental-Labors importiert und in die natürliche Ursprungssituation des Patienten in den neuen Zahnersatz überführt werden.

Entgegen dem in der Fachwelt vorherrschenden Vorurteil, dass ohne das Vorhandensein individueller 3D-Daten der zu ersetzenden Zahnstruktur eine identische oder nahezu identische Reproduktion von Zahnersatz ausgeschlossen ist und daher auch mögliche unangenehme oder sogar problematische Folgeerscheinungen für den Patienten nicht zuverlässig vermieden werden können, wird erfindungsgemäß erstmals auf der Basis der typischerweise vorhandenen 2D-Röntgenbilder eine weitgehend identische Reproduktion des verlorenen Zahns ermöglicht und so die Situation des Patienten spürbar verbessert.

Gemäß einer bevorzugten Ausführungsform der Erfindung wird ein Abbild der, aus den 2D-Röntgenbildern der ursprünglich vorhandenen natürlichen Zähne des Patienten gewonnenen, zu ersetzenden Zähne von dem Nutzer, insbesondere dem Zahnarzt, in einen Datenspeicher, beispielsweise eine Cloud oder auf einen Server hochgeladen. Das Verfahren beginnt somit mit dem Import des sogenannten Panoramaröntgenbilds des Patienten.

Zur Rekonstruktion des 3D-Modells aus dem 2D-Röntgenbild des Patienten kommt ein Algorithmus zur Anwendung. Dieser Algorithmus nutzt die Korrelation von Formen aus 2D-Röntgenbildern zu korrespondierenden 3D-Formen. Aus dieser Formenanalyse und Korrespondenzfindung einer Trainingsdatenmenge aus CT- und DVT-Datensätzen wird der benötigte Algorithmus angelegt und kann so fortlaufend optimiert werden.

Nach Einlesen des Panoramaröntgenbilds wird mit Hilfe dieses Algorithmus aus dem Röntgenbild ein dazugehöriges 3D-Modell als Datei, beispielsweise im Dateiformat ".stl", erzeugt. Diese .stl-Datei des Patienten wird dann vorzugsweise in einem Dentallabor von einem Zahntechniker aus dem Datenspeicher heruntergeladen und in seine CAD-CAM-Software importiert.

Gemäß einer besonders bevorzugten Variante wird in das CAD-CAM-Programm des Dentallabors zusätzlich der 3D-Modell-Datensatz des Zahnarztes nach dem Intra-Oral-Scan des Patienten als der individuelle Ist-Zustand des Patienten importiert. Das virtuell erstellte 3D-Modell und der 3D-Modell-Datensatz des Ist-Zustands werden dann insbesondere mittels eines Korrespondenzfindungsverfahren räumlich zueinander referenziert und auf diese Weise miteinander verknüpft. Dabei werden zumindest drei identische Punkte in beiden Modellabbildungen bestimmt. Anhand dieser drei identischen Punkte werden beide Modelldatensätze übereinandergelegt.

Der Zahntechniker kann mit Hilfe der Vorlage der ursprünglichen Form der Zähne und umgebenden Kieferanteile auf den Datensatz des Intra-Oralscans mit den fehlenden Zähnen oder Kieferanteilen die fehlenden Zähne, Zahnanteile oder Kieferanteile kopieren.

Dadurch kann auf der Basis einer Differenzbildanalyse der zu ersetzende Anteil als Zahnersatz abgeleitet werden, indem von dem Soll-Datensatz (Template) der Ist-Datensatz (Intra-Oral-San) subtrahiert wird. Der herzustellende Zahnersatz wird demnach nicht bereits vor der Erzeugung des 3D-Modells für den Patienten festgelegt, sondern ergibt sich im Anschluss der Modellbildung aufgrund der vergleichenden Betrachtung des entsprechenden 3D-Modells des Ist-Zustands.

Dieser neue Differenz-Datensatz spiegelt dann die verloren gegangenen, zu restaurierenden Zahn- und Kieferanteile wider und entspricht dem herzustellenden Zahnersatz. Bei Bedarf kann der Zahntechniker die ursprüngliche, von Natur aus angelegte Form noch modifizieren oder optimieren, falls der Patient oder der Zahnarzt diese Veränderung wünschen oder benötigen.

Der Zahnersatz wird dann in Form des fertigen Datensatzes in Form von CAM-Verfahren (Computer Aided Manufacturing) dem Stand der Technik entsprechend aus unterschiedlichsten Materialien gefertigt.

Die Erfindung lässt verschiedene Ausführungsformen zu. Zur weiteren Verdeutlichung wird eine davon anhand eines Ablaufdiagramms des erfindungsgemäßen Verfahrens näher dargestellt.

Das erfindungsgemäße Verfahren dient der Herstellung von Zahnersatz durch den Zahnarzt oder den Zahntechniker. Hierzu werden nicht vorhandene Informationen einer oder mehrerer ursprünglich vorhandener natürlicher Zähne rekonstruiert, sodass der Zahnersatz möglichst als genaues Abbild oder in Form einer Optimierung oder Modifizierung der natürlichen Zähne und Kieferanteile gefertigt werden kann.

Hierzu wird in hoher vorhersagbarer und reproduzierbarer Qualität durch Verwertung verfügbarer 2D-2D-Röntgenbilder der ursprünglich vorhandenen natürlichen Zähne des Patienten, insbesondere in Form früherer Panoramaröntgenbilder, der ursprünglich vorhandenen natürlichen Zähne des Patienten das virtuelle 3D-Modell für den Zahnersatz des Patienten erzeugt.

Ausgehend von den vorhandenen bzw. verfügbaren 2D-2D-Röntgenbilder der ursprünglich vorhandenen natürlichen Zähne des Patienten, welche ein Abbild der zu ersetzenden Zähne enthalten, wobei eine vollständige Bezahnung für die Rekonstruktion nicht nötig ist, werden zunächst in einem ersten Verfahrensschritt die 2D-2D-Röntgenbildern, der ursprünglich vorhandenen natürlichen Zähne des Patienten und gegebenenfalls einem aktuellen 3D-Scan des Patienten, erstellt durch einen beliebigen Intra-Oral-Scanner, sowie gegebenenfalls weiterer Meta-Daten wie Geschlecht oder Alter, einem Algorithmus zugeführt. Hierzu werden die benötigten Eingaben vorzugsweise einer skalierbaren Web-Plattform zugeführt.

Der Algorithmus basiert auf dem Training eines Deep-Learning-Models über reale und synthetisch generierte Datensätze. Dabei werden nach der Eingabe des 2D-Röntgenbilds in Form eines Pixel-Tensors in eine Auto-Encoder-basierte Deep-Learning-Architektur die synthetischen Datensätze auf der Grundlage von CT- und DVT-Datensätzen und 3D-Modellen generiert.

Sofern keine Röntgenstrahlen-Informationen im jeweiligem Trainingssatz vorhanden sind, wird über eine Computer-simulierte Bestrahlung ein künstliches 2D-Röntgenbild des jeweiligen 3D-Modells erzeugt und als zusätzlicher Trainingspunkt genutzt.

Anschließend erfolgt ein Finetuning des Deep-Learning-Models mit realen Datensätzen und die Erstellung eines 3D SDF-Volumes, welches beispielsweise mit Hilfe eines Marching Cubes Algorithmus in einen 3D-Tensor und schließlich in ein 3D-Triangle Mesh konvertiert wird, welches als STL-Datei exportiert wird.

Diese finale, rekonstruierte STL-Datei kann der Zahntechniker oder der Zahnarzt anschließend beispielsweise aus der Web-Plattform herunterladen und in eine CAD-CAM-Software importieren. In das CAD-CAM-Programm kann ebenso die aktuelle Patienten-Situation als 3D-Modell-Datensatz nach dem Intra-Oral-Scan des Patienten importiert werden.

Sowohl der rekonstruierte 3D-Datensatz als auch die aktuelle Situation des Patienten werden dort über ein Many-To-Many Point Matching-Verfahren miteinander verknüpft. Dabei werden mindestens drei gleiche Punkte in beiden Modellabbildungen bestimmt. Anhand dieser mindestens drei identischen Punkte werden beide Modelldatensätze übereinander gelegt, skaliert und transformiert.

Der Zahntechniker oder der Zahnarzt kann mit Hilfe der Vorlage der ursprünglichen Form der Zähne und umgebenden Kieferanteile auf den Datensatz des Intra-Oralscans mit den fehlenden Zähnen oder Kieferanteilen die fehlenden Zähne, Zahnanteile oder Kieferanteile kopieren. Dieser neue Datensatz spiegelt dann die verloren gegangenen, zu restaurierenden Zahn- und Kieferanteile wider. Diese Datei entspricht dem herzustellenden Zahnersatz.

Bei Bedarf kann die ursprüngliche, von Natur aus angelegte Form noch modifiziert oder optimiert werden, falls der Patient oder der Zahnarzt diese Veränderung wünscht oder diese erforderlich ist.

Der Zahnersatz wird dann auf der Basis dieses finalen Datensatzes in Form von CAM-Verfahren aus dem gewünschten Material gefertigt.

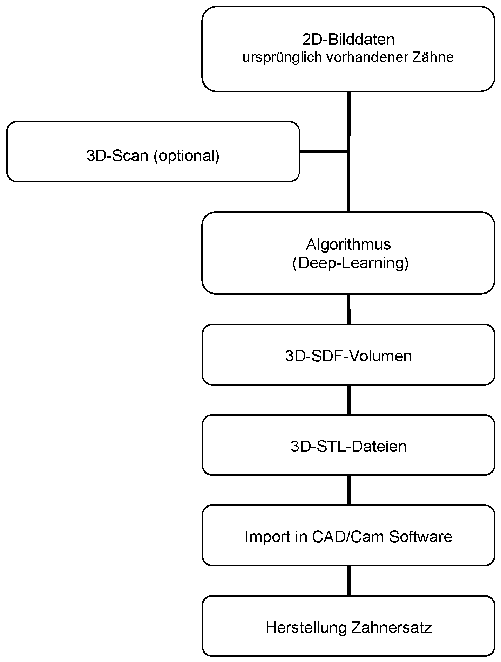

## Patentansprüche

1. Verfahren zum Visualisieren und Modellieren von Zahnersatz für einen Patienten auf der Basis zumindest eines Datensatzes eines virtuellen 3D-Modells der ursprünglich vorhandenen natürlichen Zähne des Patienten, wobei 2D-Röntgenbilder der ursprünglich vorhandenen natürlichen Zähne des Patienten, in ein Deep-Learning-Model eingegeben werden, wobei das Deep-Learning-Modell mit 2D-Röntgenbildern von Vergleichspersonen und/oder synthetisch generierten, virtuellen 2D-Röntgenbildern, und diesen 2D-Röntgenbildern zugeordneten 3D-Vergleichs-Daten zumindest einzelner Zähne oder Mundraumbereiche trainiert wurde, und wobei das Deep-Learning-Model das virtuelle 3D-Modell der ursprünglich vorhandenen natürlichen Zähne des Patienten für das Modellieren des Zahnersatzes des Patienten erzeugt.

2. Verfahren nach Anspruch 1, wobei auf der Grundlage des erzeugten virtuellen 3D-Modells der Zahnersatz und/oder ein körperliches Modell des Zahnersatzes insbesondere unter Berücksichtigung von Expertenwissen hergestellt wird.

3. Verfahren nach zumindest einem der vorhergehenden Ansprüche, **dadurch gekennzeichnet, dass** aufgrund einer vergleichenden Betrachtung eine optimale Übereinstimmung der 2D-Röntgenbilder der ursprünglich vorhandenen natürlichen Zähne des Patienten mit einzelnen 2D-Röntgenbildern von Vergleichspersonen und/oder synthetisch generierten, virtuellen 2D-Röntgenbildern ermittelt wird und die mit diesen 2D-Röntgenbildern verknüpften 3D-Vergleichs-Daten als Basis für die Erstellung des 3D-Modells für den Patienten verwendet werden.

4. Verfahren nach zumindest einem der vorhergehenden Ansprüche, **dadurch gekennzeichnet, dass** Übereinstimmungen und/oder Abweichungen der 2D-Röntgenbilder der ursprünglich vorhandenen natürlichen Zähne des Patienten und einzelner 2D-Röntgenbilder von Vergleichspersonen und/oder synthetisch generierten, virtuellen 2D-Röntgenbildern in einem automatisierten, insbesondere iterativen Prozess bestimmt werden.

5. Verfahren nach zumindest einem der vorhergehenden Ansprüche, **dadurch gekennzeichnet, dass** der Zahnersatz und/oder ein körperliches Funktionsmodell auf der Grundlage des virtuellen 3D-Modells in einem automatisierten Verfahrensschritt durch ein additives oder subtraktives Herstellungsverfahren erzeugt wird.

6. Verfahren nach zumindest einem der vorhergehenden Ansprüche, **dadurch gekennzeichnet, dass** bei der Herstellung des Zahnersatzes und/oder des körperlichen Funktionsmodells eine CAD-CAM-Software verwendet wird.

7. Verfahren nach zumindest einem der vorhergehenden Ansprüche, **dadurch gekennzeichnet, dass** ein Abbild der, aus den 2D-Röntgenbildern der ursprünglich vorhandenen natürlichen Zähne gewonnenen, zu ersetzenden Zähne in einen Datenspeicher, beispielsweise eine Cloud oder einen Server übertragen werden.

8. Verfahren nach zumindest einem der vorhergehenden Ansprüche, **dadurch gekennzeichnet, dass** das Deep-Learning-Model auf der Grundlage einer Formenanalyse und Korrespondenzfindung einer Trainingsdatenmenge aus CT- und DVT-Datensätzen erzeugt wird.

9. Verfahren nach zumindest einem der vorhergehenden Ansprüche, **dadurch gekennzeichnet, dass** das virtuell erstellte 3D-Modell und der 3D-Modell-Datensatz des Ist-Zustands insbesondere mittels eines Korrespondenzfindungsverfahren räumlich zueinander referenziert werden.

10. Verfahren nach zumindest einem der vorhergehenden Ansprüche, **dadurch gekennzeichnet, dass** ein zu ersetzender Anteil als Zahnersatz auf der Basis einer Differenzbildanalyse des virtuellen 3D-Modells und eines 3D-Datensatzes des Ist-Zustands des Patienten abgeleitet wird.

## Claims

1. Method for visualizing and modelling dentures for a patient on the basis of at least one data set of a virtual 3D model of the original natural teeth of the patient, wherein 2D X-ray images of the original natural teeth of the patient are input into a deep learning model, wherein the deep learning model was trained using 2D X-ray images of comparable persons and/or synthetically generated, virtual 2D X-ray images and 3D comparison data of at least individual teeth or oral cavity areas associated with these 2D X-ray images, and wherein the deep learning model generates the virtual 3D model of the original natural teeth of the patient for the purpose of modelling the dentures of the patient.

2. Method according to Claim 1, wherein the dentures and/or a physical model of the dentures is prepared on the basis of the generated virtual 3D model, in particular with expert knowledge being taken into account.

3. Method according to at least one of the preceding claims, **characterized in that** an optimal match between the 2D X-ray images of the original natural teeth of the patient and individual 2D X-ray images of comparable persons and/or synthetically generated, virtual 2D X-ray images is ascertained on the basis of a comparative view, and the 3D comparison data associated with these 2D X-ray images are used as the basis for the creation of the 3D model for the patient.

4. Method according to at least one of the preceding claims, **characterized in that** conformities and/or deviations of the 2D X-ray images of the original natural teeth of the patient and individual 2D X-ray images of comparable persons and/or synthetically generated, virtual 2D X-ray images are determined in an automated, in particular iterative process.

5. Method according to at least one of the preceding claims, **characterized in that** the dentures and/or a physical functional model on the basis of the virtual 3D model is generated in an automated process step by an additive or subtractive manufacturing method.

6. Method according to at least one of the preceding claims, **characterized in that** CAD-CAM software is used in the production of the dentures and/or the physical functional model.

7. Method according to at least one of the preceding claims, **characterized in that** an image of the teeth to be replaced, obtained from the 2D X-ray images of the original natural teeth, is transmitted to a data storage means, for example a cloud or a server.

8. Method according to at least one of the preceding claims, **characterized in that** the deep learning model is generated on the basis of a shape analysis and correspondence finding of a training data set from CT and DVT data sets.

9. Method according to at least one of the preceding claims, **characterized in that** the virtually created 3D model and the 3D model data set of the actual state are spatially referenced to each other, in particular by means of a correspondence finding method.

10. Method according to at least one of the preceding claims, **characterized in that** a portion to be replaced as dentures is derived on the basis of a difference image analysis of the virtual 3D model and a 3D data set of the actual state of the patient.

## Revendications

1. Procédé de visualisation et de modélisation d'une prothèse dentaire d'un patient sur la base d'au moins un jeu de données d'un modèle virtuel 3D des dents naturelles initialement présentes du patient, des images radiographiques 2D des dents naturelles initialement présentes du patient étant introduites dans un modèle d'apprentissage profond, le modèle d'apprentissage profond étant entraîné à partir d'images radiographiques 2D de personnes comparatives et/ou d'images radiographiques 2D virtuelles générées de manière synthétique, et des données de comparaison 3D associées à ces images radiographiques 2D d'au moins certaines dents ou zones de la cavité buccale, et le modèle d'apprentissage profond générant le modèle 3D virtuel des dents naturelles initialement présentes du patient pour modéliser la prothèse dentaire du patient.

2. Procédé selon la revendication 1, dans lequel la prothèse dentaire et/ou un modèle physique de la prothèse dentaire est/sont produit(s) sur la base du modèle 3D virtuel généré, en particulier en tenant compte de connaissances d'experts.

3. Procédé selon au moins l'une des revendications précédentes, **caractérisé en ce que**, sur la base d'une observation comparative, une correspondance optimale des images radiographiques 2D des dents naturelles initialement présentes du patient avec des images radiographiques 2D individuelles de personnes comparatives et/ou des images radiographiques 2D virtuelles générées de manière synthétique est établie, et les données de comparaison 3D liées à ces images radiographiques 2D sont utilisées comme base pour créer le modèle 3D pour le patient.

4. Procédé selon au moins l'une des revendications précédentes, **caractérisé en ce que** des concordances et/ou des divergences des images radiographiques 2D des dents naturelles initialement présentes du patient et des images radiographiques 2D individuelles de personnes comparatives et/ou des images radiographiques 2D virtuelles générées de manière synthétique sont déterminées lors d'un processus automatisé, en particulier itératif.

5. Procédé selon au moins l'une des revendications précédentes, **caractérisé en ce que** la prothèse dentaire et/ou un modèle fonctionnel physique est généré sur la base du modèle 3D virtuel lors d'une étape de procédé automatisée par un procédé de fabrication additif ou soustractif.

6. Procédé selon au moins l'une des revendications précédentes, **caractérisé en ce qu'**un logiciel de CAD-CAM est utilisé lors de la fabrication de la prothèse dentaire et/ou du modèle fonctionnel physique.

7. Procédé selon au moins l'une des revendications précédentes, **caractérisé en ce qu'**une image des dents à remplacer, obtenue à partir des images radiographiques 2D des dents naturelles initialement présentes, est transmise à une mémoire de données, par exemple un nuage ou un serveur.

8. Procédé selon au moins l'une des revendications précédentes, **caractérisé en ce que** le modèle d'apprentissage profond est généré sur la base d'une analyse de forme et d'une correspondance d'un jeu de données d'apprentissage composé de jeux de données CT et DVT.

9. Procédé selon au moins l'une des revendications précédentes, **caractérisé en ce que** le modèle 3D créé virtuellement et le jeu de données du modèle 3D de l'état réel sont référencés spatialement l'un par rapport à l'autre, en particulier au moyen d'un procédé de recherche de correspondance.

10. Procédé selon au moins l'une des revendications précédentes, **caractérisé en ce qu'**une partie à remplacer sous forme de prothèse dentaire est dérivée sur la base d'une analyse d'image de différence du modèle virtuel 3D et d'un jeu de données 3D de l'état réel du patient.
